# EUROPÄISCHE PATENTSCHRIFT

(11) **EP 3 621 547 B1**
(45) Veröffentlichungstag und Bekanntmachung des Hinweises auf die Patenterteilung: **10.03.2021**
(21) Anmeldenummer: 18723808.4
(22) Anmeldetag: 08.05.2018
(51) Int. Cl.: A61B 50/20, A61L 2/00, A61B 50/33, A61B 50/34, A61L 2/26

(54) **ELASTISCHE MONTAGEKLEMME**
ELASTIC MOUNTING CLAMP
ÉLASTIQUE BRIDE DE FIXATION

(30) Priorität: 08.05.2017 DE 102017109869
(43) Veröffentlichungstag der Anmeldung: 18.03.2020
(73) Patentinhaber: Aesculap AG, 78532 Tuttlingen (DE)
(72) Erfinder: BURMEISTER, Christoph, 78224 Singen (DE)
(74) Vertreter: Winter, Brandl, Fürniss, Hübner, Röss, Kaiser, Polte - Partnerschaft mbB
(86) Internationale Anmeldenummer: PCT/EP2018/061873
(87) Internationale Veröffentlichungsnummer: WO 2018/206579

(56) Entgegenhaltungen:
- US-A1- 2012 085 720
- US-A1- 2013 319 888
- US-B1- 6 436 357
- US-B2- 8 069 998

## Beschreibung

Die Erfindung betrifft eine Befestigungseinrichtung/elastische Montageklemme, welche durch eine Federkraftbeaufschlagung in Formschluss mit der Perforation bzw. den Durchbrechungen eines Sterilisationskorbes gehalten wird und an welcher eine Lagerungseinrichtung/Halterung bzw. ein Aufnahmeelement zur Anordnung/Halterung von zu sterilisierendem Gut im Korbinnenraum eines Sterilisationskorbes befestigt werden kann.

Die Erfindung betrifft zudem eine Halterungsvorrichtung mit einer solchen Lagerungseinrichtung, welche mittels einer oder mehrerer Befestigungseinrichtungen an einem Sterilisationssiebkorb befestigbar ist.

Die Erfindung betrifft außerdem ein modulares Siebkorbsystem mit einem Sterilisationssiebkorb, welcher sich nach Wunsch mit einer Anzahl an verschiedenen Lagerungseinrichtungen für verschiedenes Sterilisiergut bestücken lässt.

### Hintergrund der Erfindung

Oben beschriebene Siebkörbe, die zusätzlich einen Deckel umfassen können, finden beispielsweise in Krankenhäusern, in Kliniken, Labors oder ähnlichen Einrichtungen zur Sterilisation von entsprechenden Gütern/Instrumenten Verwendung. Darüber hinaus können die Siebkörbe zur Lagerung, zum sicheren Transport, zur Organisation oder dergleichen von den Gütern/Instrumenten eingesetzt werden. Die in einem Siebkorb eingelegten Güter werden zur Sterilisation samt dem Siebkorb in einen Sterilisator gegeben.

### Stand der Technik

Aus dem Stand der Technik sind zum Beispiel Sterilisationssiebkörbe mit Befestigungseinrichtungen und Halterungsvorrichtungen, wie sie in der DE 10 2004 008 455 B3 und der DE 20 2005 016 771 U1 gezeigt werden, bekannt. Die dort offenbarten gattungsgemäßen Halterungsvorrichtungen bestehen im Wesentlichen aus einem Steg, welcher zumindest eine Aufnahme für ein zu sterilisierendes Gut aufweist und über ein Befestigungssystem lösbar mit dem Siebkorb befestigbar ist. Die Halterungsvorrichtungen dienen als Halterung für diverse medizintechnische Güter, in welche, in der gezeigten Ausführungsform, in etwa stabförmiges Operationsbesteck im Wesentlichen quer zur Längserstreckung der Stege in diese eingesteckt werden kann. Insbesondere beim Transport des Siebkorbes kann dieser Erschütterungen ausgesetzt sein. Bei diesem Stand der Technik kann das Sterilisiergut zum einen aus der Halterungsvorrichtung herausgleiten und zum anderen kann es durch stärkere Erschütterungen Schaden nehmen. Zudem sind die Lagerungseinrichtungen bzw. Stege in dieser Offenbarung an den Seitenwänden des Sterilisationskorbes befestigt. Bei einer solchen Lösung müssen die Halterungsvorrichtungen in ihrer Länge an die Abmessungen des Siebkorbes angepasst werden und es ist oft vonnöten, dass der Steg der Halterung sich über die gesamte Breite des Siebkorbs erstreckt, um eine ausreichende Stabilität durch eine beidseitige Befestigung bereitzustellen. Dies hat zur Folge, dass jede Halterungsvorrichtung individuell für das jeweilige Set bzw. den jeweiligen Siebkorb konstruiert und produziert werden muss, was einen erheblichen Aufwand bedeutet und gegen ein nachträgliches Verändern der Siebkorbkonfiguration spricht.

Es sind aus dem Stand der Technik auch Halterungsvorrichtungen mit Befestigung in den Seitenwänden bekannt, bei denen die Stege durch Verschweißen oder Vernieten fest mit dem Siebkorb verbunden werden, was zusätzlich zu den oben beschriebenen Nachteilen ein nachträgliches Verändern der Anordnung im Siebkorb verhindert.

Weiter sind aus dem Stand der Technik Befestigungssysteme zum Befestigen der einzelnen Stege im Boden des Siebkorbs bekannt. Dabei nutzen bereits bekannte Lösungen Schraube/Mutter-Verbindungen oder Befestigungspins, um die Stege, bspw. mittels an unteren Abschnitten derselben vorgesehenen Füßen oder Biegungen, im Boden zu verankern. Derartige Systeme haben den Nachteil, vergleichsweise aufwendig in ihrer Montage und Demontage zu sein und ohne Prägung der Stege oder andere konstruktive Maßnahmen nur eine vergleichsweise geringe Steifigkeit zu bieten, was insbesondere bei der Aufnahme von schwerem Instrumentarium, wie zum Beispiel orthopädischen Instrumenten, von Nachteil sein kann. Zudem braucht man zum Lösen der Verbindungselemente solcher aus dem Stand der Technik bekannter Lösungen in der Regel Zugriff auf die Unterseite des Siebkorbs, z.B. um eine Mutter zu lösen oder den Formschluss eines Befestigungspins aufzuheben, was zum einen nicht immer möglich und zum anderen nicht ergonomisch ist.

US 2012/085720 A1 offenbart eine Befestigungseinrichtung gemäß der Präambel.

### Kurzbeschreibunq der Erfindung

Angesichts des vorstehend genannten Standes der Technik ist es die Aufgabe der vorliegenden Erfindung, eine gattungsgemäße Befestigungseinrichtung zum Befestigen einer Lagerungseinrichtung, zum Lagern bzw. Anordnen/Halten von zu sterilisierenden Teilen an einem Siebkorb, bereitzustellen, welche es erlaubt, die Konfiguration der Halterungsvorrichtungen im Siebkorb flexibel anzupassen bzw. eine individuelle Bestückung eines Siebkorbs auch mit Halterungsvorrichtungen in Stegform ermöglicht und für verschiedene Siebkorbvarianten einsetzbar ist.

Weiter macht es sich die Erfindung zur bevorzugten Aufgabe, ein Austauschen der Befestigungseinrichtungen sowie der Lagerungseinrichtungen auch im Nachhinein zuzulassen. Insbesondere ist es ein bevorzugtes Ziel, dass ein solcher Austausch auch dann möglich ist, wenn der Anwender lediglich von oben Zugriff auf den Siebkorb hat, weil der Siebkorb sich bspw. bereits im Sterilisator befindet und ein Zugriff an die Unterseite des Korbes dadurch verhindert wird. Zudem ist es ein weiteres bevorzugtes Ziel der Erfindung, dass die Befestigungseinrichtung werkzeuglos und unter geringem Platzbedarf am Behälter befestigbar ist und die Lagerungseinrichtungen werkzeuglos und unter geringem Platzbedarf an einer oder mehreren Befestigungseinrichtungen befestigbar sind. Ein weiteres bevorzugtes Ziel der vorliegenden Erfindung ist es, Befestigungseinrichtungen bereitzustellen, welche den Halterungsvorrichtungen eine ausreichende Stabilität/Steifigkeit geben, auch wenn diese gegebenenfalls nur mit einer einzelnen Befestigungseinrichtung am Boden fixiert werden. Insbesondere macht es sich die vorliegende Erfindung dabei zur bevorzugten Aufgabe, dass jede einzelne Befestigungseinrichtung per se im befestigten Zustand eine ausreichende Stabilität gegenüber einem Verkippen der Befestigungseinrichtung bereitstellt.

Die vorstehend genannten Aufgaben und Ziele werden durch eine gattungsgemäße Befestigungseinrichtung mit den im Patentanspruch 1 angegebenen Merkmalen, eine gattungsgemäße Halterungsvorrichtung mit den im Patentanspruch 12 angegebenen Merkmalen und ein gattungsgemäßes Siebkorbsystem mit den im Patentanspruch 13 angegebenen Merkmalen gelöst. Vorteilhafte Weiterbildungen der Befestigungseinrichtung sind Gegenstand der Unteransprüche.

Gemäß einem ersten Aspekt der Erfindung wird demzufolge eine Befestigungseinrichtung bzw. elastische Montageklemme zum Befestigen einer Lagerungseinrichtung für steriles oder zu sterilisierendes Gut (z.B. chirurgische Instrumente oder Implantate) in einem medizintechnischen Behälter, insbesondere einem Sterilisierkorb, vorgeschlagen.

Hierzu weist die Befestigungseinrichtung einen Lagerungs-/Aufnahmesockel (16) auf zur Lagerung/Aufnahme der Sterilgut-Halterung (11), der einen in eine erste Richtung wirkenden Hinterschneidungsabschnitt bzw. einen ersten Befestigungsabschnitt als ein Festlager hat, welcher dafür ausgebildet ist, mit einer entsprechenden Hinterschneidung am medizintechnischen Behälter in Rasteingriff gebracht zu werden.

Zudem weist die Befestigungseinrichtung ein am Lagerungs-/Aufnahmesockel direkt oder indirekt beweglich gehaltenes Hinterschneidungselement bzw. einen zweiten Befestigungsabschnitt auf als ein Loslager, das in eine entgegen der Wirkrichtung des Hinterschneidungsabschnitts gerichtete zweite Richtung wirkt und dafür ausgebildet ist, mit einer entsprechenden Hinterschneidung am medizintechnischen Behälter in Rasteingriff gebracht zu werden und mittels einem Federelement oder einem Federabschnitt am Hinterschneidungselement oder am Lagerungs-/Aufnahmesockel unter direkter oder indirekter Abstützung am Lagerungs-/Aufnahmesockel in die zweite Richtung vorspannbar ist. Dies hat den Vorteil, dass die erfindungsgemäße Befestigungseinrichtung werkzeuglos am Behälter fixiert und wieder von diesem gelöst werden kann und zum Fixieren keine weiteren Befestigungselemente, wie z.B. Schrauben, Muttern oder Befestigungspins nötig sind.

Der erste Befestigungsabschnitt ist dazu ausgebildet, einen Rand einer ersten Ausnehmung/Durchbrechung im Boden des Behälters, bzw. einen die erste Ausnehmung begrenzenden Wandungsabschnitt, formschlüssig zu umgreifen. Zudem ist der zweite Befestigungsabschnitt dazu ausgebildet, den Rand der ersten Ausnehmung und/oder einen Rand zumindest einer zweiten Ausnehmung von einer entgegengesetzten Richtung formschlüssig zu umgreifen. Anders ausgedrückt können der erste und der zweite Befestigungsabschnitt in einem Zustand, in dem die Befestigungseinrichtung am Behälter befestigt ist, jeweils von entgegengesetzten Richtungen aus, die Ränder von im Boden des Behälters vorgesehenen Ausnehmungen umgreifen, um so einen haltenden Formschluss zu schaffen. Um die Befestigungseinrichtung aus einem am Behälter befestigten Zustand in einen vom Behälter gelösten Zustand überführen zu können und umgekehrt, ist vorzugsweise zumindest einer der beiden Befestigungsabschnitte federelastisch ausgebildet, sodass unter elastischem Verformen des zumindest einen federelastischen Befestigungsabschnitts der Formschluss der Befestigungseinrichtung mit dem Behälterboden gelöst werden kann bzw. die Befestigungseinrichtung im elastisch verformten Zustand in Ausnehmungen des Behälterbodens einsetzbar ist und nach dem Einsetzen durch die federelastischen Rückstellkräfte im Formschluss mit dem Boden gehalten/geklemmt wird. Eine solche Ausführungsform, die über Löcher im Boden des Behälters festgelegt wird, hat den Vorteil, eine flexiblere Anordnung der Befestigungseinrichtung und damit der Halterung im Behälter zu ermöglichen.

Gemäß einer weiter bevorzugten Ausführungsform kann ein Halteabschnitt am Lagerungs-/Aufnahmesockel vorgesehen sein, welcher dazu ausgebildet ist, die Sterilgut-Halterung formschlüssig zu halten. Dies hat den Vorteil, dass ein werkzeugloses Fixieren, Lösen und/oder Austauschen verschiedener Lagerungseinrichtungen, die zum Lagern/Anordnen verschiedener Sterilisiergüter angepasst sein können, an einer oder mehreren Befestigungseinrichtungen möglich ist. Bevorzugt kann der Halteabschnitt weiter derart ausgebildet sein, dass die Sterilgut-Halterung, wenn der Lagerungs-/Aufnahmesockel am Boden des Behälters befestigt ist, senkrecht zur Erstreckungsrichtung des Bodens in den Halteabschnitt eingeschoben werden kann. In anderen Worten kann die Befestigungseinrichtung derart ausgebildet sein, dass die Sterilgut-Halterung vertikal in einen am Behälterboden vormontierten Lagerungs-/Aufnahmesockel einschiebbar ist. Dies hat verschiedene Vorteile. Zum einen wird durch die vertikale Einschiebbarkeit der Sterilgut-Halterung in den Lagerungs-/Aufnahmesockel der seitliche Platzbedarf bei der Montage verringert. Zum anderen kann bei stegförmigen Sterilgut-Halterungen derselbe Lagerungs-/Aufnahmesockel Halterungen unterschiedlicher Steglängen aufnehmen. Es ist also ein Lagerungs-/Aufnahmesockel für verschiedene Sterilgut-Halterungen adaptierbar. Besonders bevorzugt kann die Befestigungseinrichtung zwei Lagerungs-/Aufnahmesockel aufweisen, die dazu ausgebildet sind, jeweils einen Endabschnitt eines Steges einer Sterilgut-Halterung aufzunehmen.

Gemäß einer weiter bevorzugten Ausführungsform kann der Halteabschnitt als Blech-Umschlag ausgebildet sein, zwischen dessen im Wesentlichen U-förmigen Schenkeln die Sterilgut-Halterung, insbesondere mit steg- bzw. plattenartigen Abschnitten, einschiebbar und halterbar ist. Dies erlaubt ein einfaches Fertigen des Halteabschnitts am Lagerungs-/Aufnahmesockel durch Blechumformen. Ein solcher U-förmiger Umschlag bietet zudem eine hohe Stabilität bei vertikaler Einschiebbarkeit. Zudem können die Enden der Öffnung der U-Form als seitlicher Anschlag für eine eingeschobene Lagereinrichtung dienen.

Zum Sichern der Lagerungseinrichtung gegen ein Herausziehen aus dem Umschlag kann vorzugsweise zusätzlich ein Stift/Bolzen vorgesehen sein, welcher in einer gemeinsamen Bohrung von Halteabschnitt und Lagerungseinrichtung angeordnet werden kann.

Gemäß einer weiter bevorzugten Ausführungsform kann das Hinterschneidungselement als ein Federelement ausgebildet sein, welches an seinem einen Endabschnitt dazu ausgebildet ist, einen Formschluss mit dem medizintechnischen Behälter einzugehen und sich mit seinem anderen Endabschnitt an der in dem Halteabschnitt gehalterten Sterilgut-Halterung abstützt und diese somit in dem Halteabschnitt festklemmt. In anderen Worten kann der zweite Befestigungsabschnitt (das Hinterschneidungselement) derart zwischen die Perforation im Behälterboden und eine im Halteabschnitt gehaltene Lagerungseinrichtung klemmbar sein, dass durch die so ausgeübte Federkraft sowohl der erste Befestigungsabschnitt (Hinterschneidungsabschnitt des Lagerungs-/Aufnahmesockels) im Formschluss mit dem Behälterboden gehalten wird, als auch die Lagerungseinrichtung im Halteabschnitt zusätzlich kraftschlüssig gehalten wird.

Gemäß einer bevorzugten Ausführungsform der Erfindung kann die Befestigungseinrichtung einen Halteabschnitt aufweisen, an welchem die Lagerungseinrichtung für das Sterilisiergut befestigt oder befestigbar ist und die beiden Befestigungsabschnitte können, vorzugsweise in der Ebene des Behälterbodens, im Wesentlichen in dieselbe Richtung vom Halteabschnitt beabstandet, in Ausnehmungen des Behälterbodens eingreifen. Dies hat den Vorteil, dass durch die örtliche Nähe der Eingriffspunkte eine erfindungsgemäße Befestigungseinrichtung dazu angepasst sein kann, mit einer Hand vom Behälterboden gelöst zu werden und zudem der Platzbedarf, der zum Anbringen der Befestigungseinrichtung am Behälterboden nötig ist, gering gehalten werden kann. Eine Beabstandung der Befestigungsabschnitte zum Halteabschnitt hat zudem den Vorteil, dass ein Hebelarm gegenüber in der Lagerungseinrichtung gelagertem Sterilisiergut vergrößert werden kann, was die Stabilität der Fixierung erhöhen kann.

Gemäß einer weiteren bevorzugten Ausführungsform können die beiden Befestigungsabschnitte in dasselbe oder in benachbarte Ausnehmungen im Behälterboden eingreifen, um den Platzbedarf, der zum Anbringen der Befestigungseinrichtung am Behälterboden nötig ist, weiter zu reduzieren. Eine solche kompakte Anordnung der Befestigungsabschnitte bzw. deren Eingriffspunkte im Behälterboden erleichtert es zudem, den Befestigungsabschnitt beliebig (in eine der vier Richtungen der Seitenwände) orientiert fixieren zu können.

Gemäß einer weiteren vorteilhaften Ausbildungsform kann zudem das auf der Lagerungseinrichtung gelagerte Sterilisiergut relativ zum Halteabschnitt in die entgegengesetzte Richtung beabstandet sein, um den durch die Beabstandung der Befestigungsabschnitte vom Halteabschnitt gebildeten Hebelarm zu nutzen. In anderen Worten kann von oben betrachtet, bei einem am Behälter befestigten Befestigungsabschnitt mit daran angeordneter Lagerungseinrichtung, der Halteabschnitt zwischen dem/den zwischen Schwerpunkt(en) des zu lagernden Sterilisierguts und den Eingriffspunkten der Befestigungsabschnitte liegen oder gar einen Symmetriepunkt oder eine Symmetrieebene dazu bilden.

Gemäß einer bevorzugten Ausbildung der Erfindung kann der erste Befestigungsabschnitt dazu ausgebildet sein, bei einem Behälter mit sieb- oder gitterförmigem Boden, wie z.B. einem Sterilisationssiebkorb, eine Strebe des gitterförmigen Bodens formschlüssig zu umgreifen. Der zweite Befestigungsabschnitt kann in einem solchen Fall dazu ausgebildet sein, dieselbe Gitterstrebe von einer entgegengesetzten Richtung formschlüssig zu umgreifen. Vorteilhafterweise können der erste und der zweite Befestigungsabschnitt die Gitterstrebe dabei so umgreifen, dass sie in einer Querschnitts-/Seitenansicht ein geschlossenes Profil bilden bzw. die Gitterstrebe in ihrem Umfang einmal komplett umschließen. Eine solche Ausbildung der Erfindung erlaubt eine noch kompaktere Ausführung der Befestigungseinrichtung.

Gemäß einer bevorzugten Ausführungsform kann der erste Befestigungsabschnitt als Haken oder U-förmiger Umschlag ausgebildet und zunächst in die erste Ausnehmung im Behälterboden einsetzbar und anschließend in eine Befestigungsposition bringbar, vorzugsweise verschiebbar, sein, in der er den Rand der ersten Ausnehmung bzw. die Gitterstrebe derart formschlüssig umgreift, dass er sich nur noch in eine Vorzugsrichtung aus dem Formschluss lösen lässt. Bei einer solchen Ausführungsform kann der zweite Befestigungsabschnitt federelastisch und dazu angepasst sein, derart mit zumindest einer weiteren Ausnehmung bzw. der Gitterstrebe in Eingriff gebracht zu werden, dass die Befestigungseinrichtung mit einer Federkraft entgegen der Vorzugsrichtung beaufschlagt wird.

Gemäß einer bevorzugten Ausbildung der Erfindung kann der zweite Befestigungsabschnitt einen Formschluss quer zur Vorzugsrichtung herstellen, indem er im befestigten Zustand an zumindest zwei Rändern von Ausnehmungen im Boden anliegt, vorzugsweise zwei Ränder, welche im Wesentlichen parallel zur Vorzugsrichtung verlaufen. Gemäß einer weiteren bevorzugten Ausführungsform kann der zweite Befestigungsabschnitt dazu gabelförmig ausgebildet sein und zwei Ränder von Ausnehmungen im Boden formschlüssig zwischen seinen Gabelzinken formschlüssig halten oder zwischen zwei Rändern von Ausnehmungen im Boden formschlüssig gehalten werden, um einen Formschluss quer zur Vorzugsrichtung herzustellen. Auf diese Weise lässt sich unter geringem Platzbedarf ein Spiel der Befestigungseinrichtung quer zur Vorzugsrichtung (entlang des vom ersten Befestigungsabschnitt umgriffenen Rands/Gitterstrebe) minimieren. Vorzugsweise kann der zweite Befestigungsabschnitt bei einer solchen gabelförmigen Ausführungsform den ersten Befestigungsabschnitt seitlich umgreifen, um Platz zu sparen und Ausnehmungen bzw. die Gitterstrebe, ähnlich einem Gabelstapler, untergreifen, um einen zusätzlichen Formschluss zu schaffen.

Gemäß einer bevorzugten Ausführungsform der Erfindung sind sowohl der erste als auch der zweite Befestigungsabschnitt im befestigten Zustand an der Behälterinnenseite angeordnet und greifen von der Behälterinnenseite aus in die Ausnehmung(en) ein, sodass die Befestigungseinrichtung ausschließlich durch Zugriff von der Behälterinnenseite aus, d.h. ohne dass ein Zugriff von einer Behälterunterseite aus nötig ist, befestigbar und lösbar ist.

Gemäß einer weiteren bevorzugten Ausbildung kann die Befestigungseinrichtung eine Auflagefläche bilden, mit welcher sie auf dem Behälterboden aufliegt, wobei bevorzugt der Halteabschnitt an einem Ende der Auflagefläche liegen kann und die Befestigungsabschnitte am gegenüberliegenden Ende der Auflagefläche in die Perforation des Behälterbodens eingreifen können. Durch eine solche Auflagefläche kann die Stabilität der Befestigungseinrichtung gegenüber einer Verkippbewegung erhöht werden.

Bevorzugt kann die Auflagefläche dabei eine Verlängerung bilden, die über den Eingriffspunkt des ersten und/oder zweiten Befestigungsabschnitts in den Behälterboden hinausragt und dazu angepasst ist, einen gegenüber einem Verkippen der Befestigungseinrichtung abstützenden Kontakt mit dem Behälterboden herzustellen.

Der zweite Befestigungsabschnitt kann gemäß einer bevorzugten Ausführungsform lösbar mit der Befestigungseinrichtung verbunden sein. Dabei wird er bevorzugt über einen Stift (bzw. den Stift, der die Lagerungseinrichtung im Formschluss hält) formschlüssig und über seine internen elastischen Rückstellkräfte kraftschlüssig an der Befestigungseinrichtung gehalten. Eine modulare Ausführung des zweiten Befestigungsabschnitts kann das Befestigen und Lösen der Befestigungseinrichtung mit dem Boden erleichtern und ermöglicht einen Austausch des zweiten Befestigungsabschnitts, z.B. bei einem Defekt oder um ihn an eine andere Gitterstruktur einer anderen Siebkorbvariante anpassen zu können.

Gemäß einer bevorzugten Ausbildung kann die Befestigungseinrichtung aus einem medizinisch zugelassenen Material mit einer hohen Belastbarkeit gegenüber wechselnden Temperatur- und Druckbelastungen gefertigt sein, vorzugsweise einem medizintechnischen Edelstahl oder Aluminium. Der zweite Befestigungsabschnitt besteht dabei vorzugsweise aus einem medizintechnischen Federstahl.

Gemäß einem weiteren Aspekt der Erfindung ist eine Halterungsvorrichtung vorgesehen mit einem oder mehreren erfindungsgemäßen Befestigungsabschnitten, an welchen eine oder mehrere Lagerungseinrichtungen zum Lagern/Anordnen von Sterilisiergut angeordnet werden können. Dies hat den Vorteil, dass für verschiedene Sterilisiergüter, wie z.B. chirurgische Instrumente, angepasste Lagerungseinrichtungen entwickelt/gefertigt werden können, die alle dazu ausgebildet sind, von einer oder mehreren erfindungsgemäßen Befestigungseinrichtungen aufgenommen zu werden bzw. an einer oder mehreren Befestigungseinrichtungen befestigbar zu sein. D.h. dass eine erfindungsgemäße Halterungsvorrichtung es erlaubt, mit wenigen Varianten an Befestigungseinrichtungen eine Vielzahl an verschiedenen Lagerungseinrichtungsvarianten für diverse Sterilisiergüter, flexibel und werkzeuglos an medizintechnischen Behältern zu fixieren.

Ein weiterer Aspekt der Erfindung betrifft ein modulares Siebkorbsystem zum Anordnen/Lagern von sterilem oder zu sterilisierendem Gut mit
- einem Siebkorb, insbesondere einem Sterilisationssiebkorb, mit einem durch einen Boden und Seitenwände definierten Innenraum und einer Anzahl an Ausnehmungen im Boden; und
- einer Anzahl an Halterungsvorrichtungen, insbesondere Halterungsvorrichtungen gemäß Anspruch 9, welche mit den Ausnehmungen in Eingriff bringbar und modular aus Lagerungseinrichtungen und Befestigungseinrichtungen, insbesondere Befestigungseinrichtungen gemäß einem der Ansprüche 1 bis 8, aufgebaut sind. Dabei können erfindungsgemäß die Befestigungseinrichtungen flexibel mit der Perforation des Siebkorbbodens in Eingriff bringbar und dazu angepasst sein, unterschiedliche Lagerungseinrichtungen aufzunehmen.

Gemäß einer vorteilhaften Weiterbildung kann die Perforation verschiedener Siebkörbe so ausgebildet sein, dass erfindungsgemäße Befestigungseinrichtungen siebkorbübergreifend, d.h. für Siebkörbe verschiedener Systeme und mit verschiedenen Abmessungen, einsetzbar sind.

### Fiqurenbeschreibunq

Die Erfindung wird nachstehend anhand bevorzugter Ausführungsbeispiele unter Bezugnahme auf die begleitenden Figuren näher erläutert.

### Es zeigen

Fig. 1 eine perspektivische Ansicht eines Siebkorbes mit an dessen Seitenwänden festgelegten Halterungsvorrichtungen gemäß dem Stand der Technik;
Fig. 2 eine Rück- und eine Vorderansicht einer erfindungsgemäßen Halterungsvorrichtung mit einer erfindungsgemäßen Befestigungseinrichtung, welche im perforierten Boden eines Siebkorbes fixiert ist;
Fig. 3 eine Nahaufnahme einer erfindungsgemäßen Befestigungseinrichtung;
Fig. 4 eine Ansicht einer erfindungsgemäßen Halterungsvorrichtung von unten, im am Siebkorb fixierten Zustand.

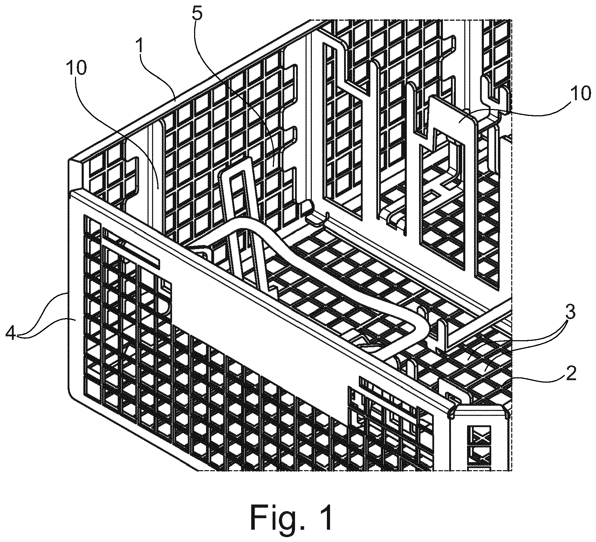

In Fig.1 ist ein Siebkorb 1 dargestellt, wie er in Sterilisierbehältern zum Sterilisieren von chirurgischen Elementen und dergleichen als Einsatz verwendet wird. Der siebartige Boden 2 weist eine Vielzahl von Ausnehmungen/Durchbrechungen 3 auf.

Die in Fig. 1 dargestellten Halterungsvorrichtungen 10 entsprechen dem Stand der Technik und sind an den Seitenwänden des Siebkorbs 1 an demselben festgelegt, im gezeigten Beispiel durch Verschweißen der seitlichen Stirnabschnitte der stegartigen Halterungsvorrichtungen 10 mit den Seitenwänden 4 des Siebkorbs 1. Die Halterungsvorrichtungen 10 können eine Vielzahl von Lagerungseinrichtungen 11 zum Anordnen/Lagern von diversen chirurgischen Instrumenten und sonstigem Sterilisiergut aufweisen. Diese können im Grunde beliebig an die Geometrie des anzuordnenden bzw. zu lagernden Sterilisierguts angepasst sein und beispielsweise Ausnehmungen und/oder Auskragungen bzw. Trägervorrichtungen ausbilden. Die gezeigte Ausführungsform mit Fixierung der Halterungsvorrichtungen 10 in den Seitenwänden 4 des Siebkorbs bringt den Nachteil mit sich, dass sich die Halterungsvorrichtungen 10 in der Regel von einer Seitenwand 4 des Siebkorbs 1 zur Gegenüberliegenden erstrecken und deshalb ein Verwenden einer solchen Halterung 10 mit einem Siebkorb 1 mit anderen Maßen nicht möglich ist. Dies hat zur Folge, dass oftmals für jede neue Siebkorbvariante individuell neue Halterungsvorrichtungen 10 konstruiert und gefertigt werden müssen.

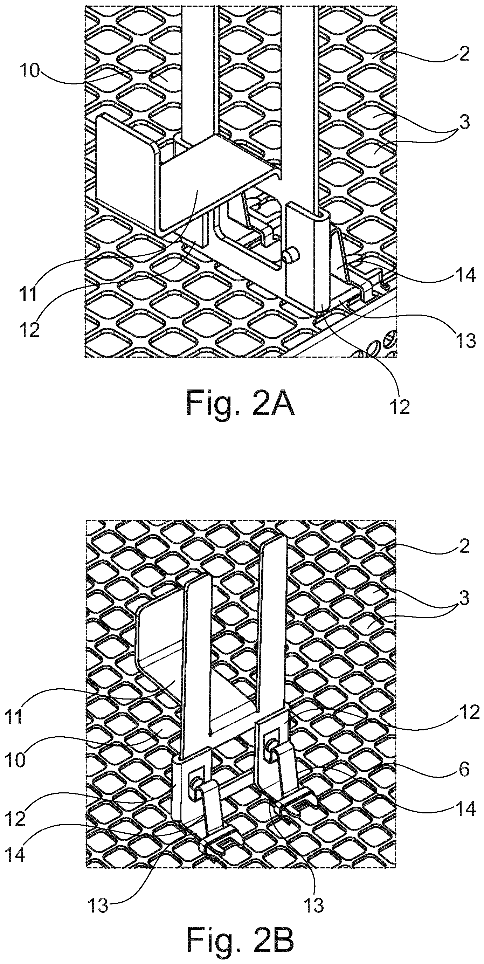

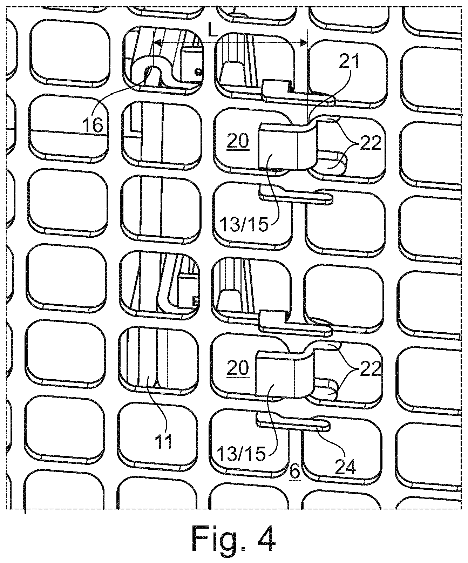

Die in den Figuren 2 bis 4 gezeigte erfindungsgemäße, mittels Befestigungseinrichtungen 12 befestigte, Halterungsvorrichtung 10 bzw. ein erfindungsgemäßes Siebkorbsystem macht es sich aufgrund dieser und anderer aus dem Stand der Technik bekannter Nachteile zur bevorzugten Aufgabe, ein modulares Befestigungssystem bereitzustellen, bei dem die Halterungsvorrichtungen 10 mittels Befestigungseinrichtungen 12, mit geringem Aufwand werkzeuglos lösbar, am Boden des Siebkorbs 1 fixiert werden und welches im Folgenden anhand einer bevorzugten Ausführungsform genauer beschrieben wird. Auf diese Weise lassen sich für einzelne Produkte/Produktgruppen Lagerungseinrichtungen 11 fertigen und ans Lager legen. Diese können durch die erfindungsgemäßen Befestigungseinrichtungen 12 schnell, einfach und modular zu einem Set zusammenstellen.

Grundsätzlich weist eine erfindungsgemäße Halterungsvorrichtung 10 gemäß dem gezeigten Ausführungsbeispiel eine Lagerungseinrichtung 11 und eine Anzahl (hier zwei) von Befestigungseinrichtungen 12 auf. Die Lagerungseinrichtungen 11 können, wie aus dem Stand der Technik bekannt, eine Vielzahl an Ausführungsformen annehmen, individuell an das darauf zu lagernde Sterilisiergut angepasst werden und müssen erfindungsgemäß lediglich dazu angepasst sein, direkt oder indirekt von einer oder mehreren Befestigungseinrichtungen 12 aufgenommen bzw. an diesen angeordnet zu werden. Die gezeigten Befestigungseinrichtungen 12 sind dazu angepasst, in beliebige benachbarte Ausnehmungen 3 des dargestellten, siebartigen/perforierten Bodens 2 einzugreifen (eine beliebige Gitterstrebe zu umgreifen) und können theoretisch, in eine beliebige Raumrichtung ausgerichtet, an diesem fixiert werden. Die gezeigte Ausführungsform erlaubt es also, mit einer relativ geringen Anzahl von Befestigungseinrichtungsvarianten 12 (hier zwei, eine links und rechts für jede Stirnseite der hier stegartig ausgebildeten Lagereinrichtung 11), welche nahezu beliebig am Boden 2 des Siebkorbs 1 positionierbar sind, eine Vielzahl an Ausführungsformen der Lagerungseinrichtungen 11 anzuordnen/festzulegen. Die erfindungsgemäßen Befestigungseinrichtungen 12 lassen sich mit anderen Worten also sowohl siebkorb- als auch lagerungseinrichtungsübergreifend verwenden. Darüber hinaus benötigt die gezeigte Ausführungsform der Befestigungseinrichtung 12 nur wenige (drei) benachbarte Ausnehmungen 3, um am Behälterboden 2 befestigt zu werden.

Die Befestigungseinrichtung 12 weist in der gezeigten Ausführungsform eine im Wesentlichen L-förmige Geometrie bzw. einen L-förmigen Lagersockel 16 auf. Im vertikalen Abschnitt der L-Form des Lagersockels 16 ist dabei ein Halteabschnitt 17 für eine Lagerungseinrichtung 11 vorgesehen, während der untere Abschnitt der L-Form eine Auflagefläche 20 bildet. Am distalen Ende der Auflagefläche 20 bildet die Befestigungseinrichtung 12 einen ersten Befestigungsabschnitt 13 aus, der dazu angepasst ist, einen formschlüssigen Eingriff mit dem Rand einer Ausnehmung 3 im Behälterboden 2 zu erzeugen, in diesem Beispiel durch einen in etwa U-förmigen Umschlag bzw. Haken 15 an der Unterseite des distalen Endes der Auflagefläche 20. Durch die U-Form des ersten Halteabschnitts 13 und die perforierte Siebstruktur des Bodens 2 lässt sich die Befestigungseinrichtung 12, nachdem sie mit der Ausnehmung 3 in Eingriff gebracht wurde, nur noch durch eine Bewegung in einer Vorzugsrichtung aus diesem Eingriff lösen. Ein zweiter Befestigungsabschnitt 14 der Befestigungseinrichtung 12 ist im gezeigten Beispiel als Federelement aus Federblech ausgeführt, welches zwischen die Vorderseite bzw. die dem Fuß zugewandte Seite des vertikalen Abschnitts der L-Form und gegenüberliegende Ränder von Ausnehmungen 3 im Boden 2 geklemmt/gespannt ist, sodass die Befestigungseinrichtung 12 entgegen der Vorzugsrichtung gedrückt und somit mit der Perforation 3 verankert wird. Der Eingriff des zweiten Befestigungsabschnitts 14 mit der Perforation 3 des Bodens 2 ist dabei im gezeigten Beispiel konstruktiv so ausgeführt, dass sich das Federblech in distaler Richtung aufgabelt und zwei Gabelzinken 24 bildet, welche links und rechts zur Auflagefläche 20 benachbarte Ausnehmungen 3 untergreifen und somit zusätzlich zu der Klemmwirkung mit den Rändern der Perforation 2 einen weiteren Formschluss, entgegengesetzt zur Richtung des durch den ersten Befestigungsabschnitt 13 erzeugten Formschlusses, erzeugen. Die gezeigte Befestigungseinrichtung 12 ist durch die genannten konstruktiven Merkmale dazu ausgebildet, in wenige benachbarte Ausnehmungen 3 eingreifen und dennoch eine vergleichsweise hohe Stabilität bieten zu können. Die Gabelzinken haben in der gezeigten Ausführungsform zudem die Funktion, dass sie zwei Gitterstreben 6 bzw. zwei parallel zur Vorzugsrichtung verlaufende Ränder von Ausnehmungen 3 umgreifen bzw. zwischen sich aufnehmen und dadurch einen Formschluss quer zur Vorzugsrichtung schaffen.

Ein bevorzugtes Ziel der gezeigten, erfindungsgemäßen Halterung ist es, eine vergleichsweise hohe Stabilität bzw. ein vergleichsweise geringes Spiel gegenüber einem Verkippen der Befestigungseinrichtung im fixierten Zustand bereitzustellen. Zu diesem Zweck sind der Halteabschnitt 17 für die Lagerungseinrichtung 11 und der erste Befestigungsabschnitt 13 an entgegengesetzten Enden der Auflagefläche 20 angeordnet und somit in einer Ebene parallel zu der des Bodens 2 zueinander beabstandet (L), wodurch die Stabilität gegen ein Verkippen entlang der proximalen Kante der L-Form der Befestigungseinrichtung erhöht wird. Insbesondere erhöht sich durch diese Beabstandung (L) auch der Hebelarm der Lagerkraft an einem Eingriffspunkt 21, an welchem der erste Befestigungsabschnitt 13 in die Perforation 3 des Bodens 2 eingreift, gegenüber einem vorzugsweise (mit seinem Schwerpunkt) auf der entgegengesetzten Seite der Halterung gelagerten Sterilisiergut.

Ein Verkippen in die andere Richtung, d.h. über die distale Endkante der L-Form der Befestigungseinrichtung 12, wird konstruktiv dadurch minimiert, dass sich die Auflagefläche 20 über den Eingriffspunkt 21 hinaus gabelzinkenartig verlängert, wodurch sich die Endkante der Auflagefläche 20 in distale Richtung verlagert und die distalen Gabelzinken 24 des zweiten Befestigungsabschnitts 14 durch das Untergreifen der benachbarten Ausnehmungen 3 ein Gegenlager zu einer solchen Kippbewegung bilden. Die besagten Gabelzinken 24 des Federblechs 4 erzeugen zudem dadurch, dass sie in die jeweils quer zur Längsrichtung der Auflagefläche 20 mit dieser benachbarten Ausnehmungen 3 eingreifen, eine Stabilität gegenüber einem Verkippen in ebenjener Querrichtung.

Wie in Fign. 2A und 2B gut zu erkennen ist, weisen die Befestigungseinrichtungen 12 Halteabschnitte 17 auf, welche in ihrem Querschnitt im Wesentlichen U-förmig als Umschlag ausgebildet sind und von deren U-förmigen Schenkeln die Stirnseiten der steg- bzw. plattenartigen Unterseite der Lagerungseinrichtung 11 umgriffen bzw. formschlüssig gehalten wird. Der so erzeugte Formschluss kann die Steifigkeit/Stabilität der Verbindung zwischen Lagerungseinrichtung 11 und Befestigungseinrichtung 12 deutlich erhöhen und das sackartige Ende der Öffnung der U-Form kann zudem als seitlicher Anschlag dienen.

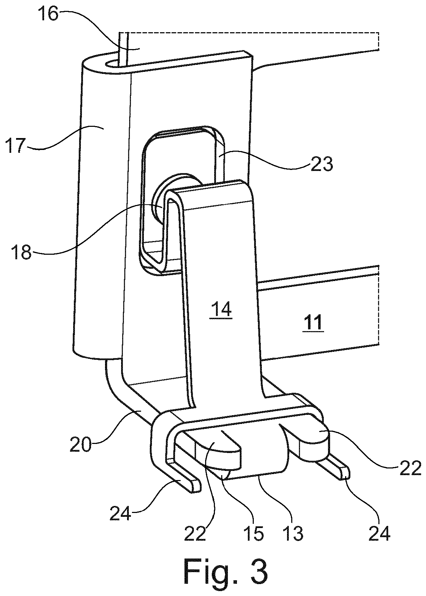

Im gezeigten Ausführungsbeispiel wird die Lagerungseinrichtung 11 zusätzlich durch eine Stift-Bohrungs-Verbindung 18, 19 mit der Befestigungseinrichtung 12 gehalten und ein Herausziehen bzw. -lösen der Lagereinrichtung aus der U-förmigen Umfassung durch die Befestigungseinrichtung 12 damit verhindert. Wie besonders in der in Fig. 3 dargestellten Detailansicht gut zu sehen ist, übt zudem der zweite Befestigungsabschnitt 14 im fixierten Zustand eine Anpresskraft auf die Lagerungseinrichtung 11 aus, indem er durch eine Ausnehmung 23 in einem U-Schenkel des Halteabschnitts 17 hindurchgreift und die Lagerungseinrichtung 11 gegen den dahinterliegenden, zweiten U-Schenkel drückt. Diese Kombination aus komplettem Formschluss durch die U-förmige Aufnahme 17 und den Stift 18 mit zusätzlichem Kraftschluss durch das Federelement 14 ermöglicht eine höhere Stabilität der Verbindung im Vergleich zu Lösungen, wie sie aus dem Stand der Technik bekannt sind.

Der zweite Befestigungsabschnitt 14 ist im gezeigten Beispiel lösbar mit der Befestigungseinrichtung 12 verbunden und wird über den Stift 18 formschlüssig und über seine internen elastischen Rückstellkräfte kraftschlüssig an der Befestigungseinrichtung gehalten. Eine modulare Ausführung des zweiten Befestigungsabschnitts 14 kann das Befestigen und Lösen der Befestigungseinrichtung 12 mit dem Boden 2 erleichtern und ermöglicht einen Austausch des zweiten Befestigungsabschnitts 14, z.B. bei einem Defekt oder um ihn an eine andere Gitterstruktur eines anderen Siebkorbs 1 anpassen zu können.

Die beispielhaft dargestellte Befestigungseinrichtung 12 ist aus einem medizintechnisch kompatiblen Metall gefertigt, es ist jedoch genauso die Verwendung eines zur Sterilisation geeigneten Kunststoffs denkbar.

Ausgehend von dem dargestellten Ausführungsbeispiel kann die erfindungsgemäße Befestigungseinrichtung 12 in vielerlei Hinsicht abgewandelt werden.

So lässt sich die Erfindung auch abseits von medizintechnischen Anwendungen als Befestigungslösung an sieb- oder gitterförmigen Oberflächen verwenden.

Auch die gezeigte Kombination von Ausnehmungen 3, in welche die Befestigungsabschnitte 13, 14 eingreifen, kann nahezu beliebig variiert werden und auch die Anzahl an Befestigungseinrichtungen 12, die eine Lagerungseinrichtung 11 befestigen, kann variiert werden, so ist es z.B. auch denkbar, dass eine einzelne Befestigungseinrichtung 12 eine Lagerungseinrichtung 11 sicher befestigt.

### Bezugszeichenliste

- 1: Behälter/Siebkorb
- 2: Boden
- 3: Ausnehmung/Perforation
- 4: Seitenwände
- 5: Behälterinnenraum/-innenseite
- 6: Gitterstrebe
- 10: Halterungsvorrichtung
- 11: Lagerungseinrichtung/(Sterilgut-)Halterung
- 12: Befestigungseinrichtung
- 13: (fester) Hinterschneidungsabschnitt/erster Befestigungsabschnitt
- 14: (bewegliches) Hinterschneidungselement/zweiter Befestigungsabschnitt
- 15: Haken/Umschlag
- 16: Lagerungssockel
- 17: Halteabschnitt/Umschlag
- 18: Stift
- 19: Bohrung
- 20: Auflagefläche
- 21: Eingriffspunkt
- 22: Verlängerung
- 23: Ausnehmung
- 24: Gabelzinken

## Patentansprüche

1. Befestigungseinrichtung (12) einer vorzugsweise stegförmigen Sterilgut-Halterung (11) zu deren Befestigung in einem medizintechnischen Behälter (1), insbesondere einem Sterilisationssiebkorb, mit:
einem Lagerungs-/Aufnahmesockel (16) zur Lagerung/Aufnahme der Sterilgut-Halterung (11), der einen in eine erste Richtung wirkenden Hinterschneidungsabschnitt (13) als ein Festlager hat, der dafür ausgebildet ist, mit einer entsprechenden Hinterschneidung am medizintechnischen Behälter (1) in Rasteingriff gebracht zu werden,
einem am Lagerungs-/Aufnahmesockel (16) direkt oder indirekt beweglich gehaltenen Hinterschneidungselement (14) als ein Loslager, das in eine entgegen der Wirkrichtung des Hinterschneidungsabschnitts gerichtete zweite Richtung wirkt und dafür ausgebildet ist, mit einer entsprechenden Hinterschneidung am medizintechnischen Behälter (1) in Rasteingriff gebracht zu werden und
einem Federelement oder einem Federabschnitt (14) am Hinterschneidungselement (14) oder am Lagerungs-/Aufnahmesockel (16), welches dafür ausgebildet ist, das Hinterschneidungselement (14) unter direkter oder indirekter Abstützung am Lagerungs-/Aufnahmesockel in die zweite Richtung vorzuspannen, **dadurch gekennzeichnet, dass**
der Hinterschneidungsabschnitt (13) U- oder hakenförmig ist und ausgebildet ist, um einen Rand einer am Boden (2) des Behälters (1) vorgesehenen ersten Ausnehmung (3) formschlüssig zu umgreifen, und
das Hinterschneidungselement (14) ausgebildet ist, einen Rand der ersten Ausnehmung (3) und/oder zumindest einer zweiten Ausnehmung (3) von einer entgegengesetzten Richtung formschlüssig zu umgreifen, wobei
zumindest einer der beiden Befestigungsabschnitte (13, 14) federelastisch ausgebildet ist.

2. Befestigungseinrichtung (12) nach Anspruch 1, **gekennzeichnet durch** einen Halteabschnitt (17) am Lagerungs-/Aufnahmesockel (16), welcher dazu ausgebildet ist, die Sterilgut-Halterung (11) formschlüssig zu halten und welcher derart ausgebildet ist, dass die Halterung (11), wenn der Lagerungs-/Aufnahmesockel (16) am Boden (2) des Behälters (1) befestigt ist, senkrecht zur Erstreckungsrichtung des Bodens (2) in den Halteabschnitt (17) eingeschoben werden kann.

3. Befestigungseinrichtung (12) nach Anspruch 2, **dadurch gekennzeichnet, dass** der Halteabschnitt (17) als Blech-Umschlag ausgebildet ist, zwischen dessen im Wesentlichen U-förmigen Schenkeln die Sterilgut-Halterung (11) einschiebbar und halterbar ist.

4. Befestigungseinrichtung (12) nach Anspruch 2 oder 3, **dadurch gekennzeichnet, dass** das Hinterschneidungselement (14) als ein Federelement ausgebildet ist, welches an seinem einen Endabschnitt dazu ausgebildet ist, einen Formschluss mit dem medizintechnischen Behälter (1) einzugehen und sich mit seinem anderen Endabschnitt an der in dem Halteabschnitt (17) gehalterten Sterilgut-Halterung (11) abstützt und diese somit in dem Halteabschnitt (17) festklemmt.

5. Befestigungseinrichtung (12) nach Anspruch 1, **dadurch gekennzeichnet, dass** der Lagersockel (16) einen Halteabschnitt (17) aufweist, an welchem die Halterung (11) befestigt oder befestigbar ist und sowohl der Hinterschneidungsabschnitt (13) als auch das Hinterschneidungselement (14), in einem Zustand, in dem die Halterung (11) mittels der Befestigungseinrichtung (12) am Behälter 1 befestigt ist, im Wesentlichen in derselben Richtung von dem Halteabschnitt (17) beabstandet in Ausnehmungen (3) im Boden (2) eingreifen.

6. Befestigungseinrichtung (12) nach Anspruch 1 oder 2, **dadurch gekennzeichnet, dass** der medizintechnische Behälter (1) einen sieb- oder gitterförmigen Boden (2) aufweist und der Hinterschneidungsabschnitt (13) dazu ausgebildet ist, um eine Gitterstrebe (6) formschlüssig zu umgreifen, während das Hinterschneidungselement (14) dazu ausgebildet ist, die Gitterstrebe (6) von einer entgegengesetzten Richtung formschlüssig zu umgreifen, insbesondere so zu umgreifen, dass der Hinterschneidungsabschnitt (13) und das Hinterschneidungselement (14) im befestigten Zustand in einer Querschnittsansicht / Projektion ein geschlossenes Profil aufweisen bzw. die Gitterstrebe (6) umschließen.

7. Befestigungseinrichtung (12) nach einem der vorgenannten Ansprüche, **dadurch gekennzeichnet, dass** der Hinterschneidungsabschnitt (13) in die erste Ausnehmung (3) einsetzbar ist und in eine Befestigungsposition bringbar, insbesondere verschiebbar ist, in welcher der Befestigungsabschnitt den Rand der ersten Ausnehmung (3) bzw. die Gitterstrebe (6) derart formschlüssig umgreift, dass sich der Hinterschneidungsabschnitt (13) nur durch eine Bewegung in eine Vorzugsrichtung aus dem Formschluss lösen lässt und das Hinterschneidungselement (14) federelastisch ausgebildet und derart mit Ausnehmungen (3) in Eingriff bringbar ist, dass die Befestigungseinrichtung (12) mit einer Federkraft entgegen der Vorzugsrichtung beaufschlagt wird.

8. Befestigungseinrichtung (12) nach Anspruch 7, **dadurch gekennzeichnet, dass** das Hinterschneidungselement (14) einen Formschluss quer zur Vorzugsrichtung herstellt, indem er im befestigten Zustand an zumindest zwei, insbesondere im Wesentlichen parallel zur Vorzugsrichtung verlaufenden, Rändern von Ausnehmungen (3) anliegt.

9. Befestigungseinrichtung (12) nach Anspruch 8, **dadurch gekennzeichnet, dass** das Hinterschneidungselement (14) gabelförmig ausgebildet ist und den Hinterschneidungsabschnitt (13) seitlich umgreift.

10. Befestigungseinrichtung (12) nach einem der vorgenannten Ansprüche, **dadurch gekennzeichnet, dass** der erste Hinterschneidungsabschnitt (13) und das Hinterschneidungselement (14) im befestigten Zustand in einer Behälterinnenseite (5) angeordnet sind und von der Behälterinnenseite (5) aus in die Ausnehmungen (3) eingreifen, wodurch die Befestigungseinrichtung (12) durch einen Zugriff ausschließlich von der Behälterinnenseite (5) aus vom Behälter (1) lösbar ist.

11. Befestigungseinrichtung (12) gemäß einem der Ansprüche 1 bis 10, **dadurch gekennzeichnet, dass** die Befestigungseinrichtung (12) eine Auflagefläche (20) bildet, welche auf dem Boden (2) des Behälters (1) aufliegt und die Auflagefläche (20) zumindest eine Verlängerung (22) bildet, welche in eine Richtung weg vom Halteabschnitt (16) über den der Hinterschneidungsabschnitt (13) hinausragt und zu einem, gegenüber einem Verkippen der Befestigungseinrichtung (12) abstützenden, Kontakt mit dem Boden (2) angepasst ist.

12. Halterungsvorrichtung (10) zur Anordnung/Lagerung von sterilem oder zu sterilisierendem Gut im Innenraum (5) eines medizintechnischen Behälters (1), insbesondere eines Sterilisationssiebkorbes, welcher einen Boden (2) mit einer Anzahl an Ausnehmungen (3) aufweist, mit
- einer Sterilgut-Halterung (11) zum Halten und/oder Lagern von sterilem oder zu sterilisierendem Gut,
- zumindest zwei Befestigungseinrichtungen (12) gemäß einem der Ansprüche 1 bis 11,
**dadurch gekennzeichnet, dass**
die Sterilgut-Halterung (11) an jeder ihrer Stirnseiten mit jeweils einer Befestigungseinrichtung (12) durch Formschluss werkzeuglos lösbar befestigbar ist, und die Befestigungseinrichtungen (12) dazu angepasst sind, verschiedene Sterilgut-Halterungen (11), welche ihrerseits jeweils zur Anordnung/Lagerung unterschiedlichen Sterilisierguts angepasst sind, aufzunehmen.

13. Modulares Siebkorbsystem zum Anordnen/Lagern von sterilem oder zu sterilisierendem Gut mit
- einem Siebkorb (1), insbesondere einem Sterilisationssiebkorb, mit einem durch einen Boden (2) und Seitenwände (4) definierten Innenraum (5) und einer Anzahl an Ausnehmungen (3) im Boden (2); und
- einer Anzahl an Halterungsvorrichtungen (10) gemäß Anspruch 12, welche mit den Ausnehmungen (3) in Eingriff bringbar und modular aus Sterilgut-Halterungen (11) und Befestigungseinrichtungen (12) gemäß einem der Ansprüche 1 bis 11 aufgebaut sind,
**dadurch gekennzeichnet, dass**
zum individuellen Bestücken des Siebkorbes (1) die Befestigungseinrichtungen (12) flexibel mit verschiedenen Ausnehmungen (3) in Eingriff bringbar und dazu angepasst sind, unterschiedliche Sterilgut-Halterungen (11) aufzunehmen/festzulegen, welche ihrerseits jeweils zur Anordnung/Lagerung unterschiedlichen Sterilisierguts angepasst sind.

## Claims

1. A securing device (12) of a preferably strut-shaped sterile product mounting (11) for securing it in a medical container (1), in particular a sterilization mesh basket, comprising:
a supporting/receiving base (16) which is provided for supporting/receiving the sterile product mounting (11) and has an undercut portion (13) as a fixed bearing acting in a first direction and designed to come into latching engagement with a corresponding undercut on the medical container (1),
an undercut element (14) as a floating bearing and movably retained in a direct or indirect fashion on the supporting/receiving base (16), said floating bearing acting in a second direction opposite the operating direction of the undercut portion and being designed to be brought into a latching engagement with a corresponding undercut on the medical container (1), and
a spring element or a spring portion (14) provided on the undercut element (14) or on the supporting/receiving base (16), which is designed to preload the undercut element (14) in the second direction while being directly or indirectly supported on the supporting/receiving base, **characterized in that**
the undercut portion (13) is U-shaped or hook-shaped and is designed to engage around an edge of a first recess (3) provided on the base (2) of the container (1) in a form-fitting manner, and
the undercut element (14) is designed to engage around an edge of the first recess (3) and/or of at least one second recess (3) in a form-fitting manner from an opposite direction, wherein
at least one of said securing portions (13, 14) is designed to be spring-elastic.

2. The securing device (12) according to claim 1, **characterized by** a holding section (17) which is provided on the supporting/receiving base (16) and designed to hold the sterile product mounting (11) in a form-fitting manner, and which is designed such that the mounting (11), if the supporting/receiving base (16) is secured on the base (2) of the container (1), can be inserted into the holding section (17) perpendicular to the extending direction of the base (2).

3. The securing device (12) according to claim 2, **characterized in that** the holding section (17) is designed as a sheet metal bent section which has substantially U-profile-shaped legs between which the sterile product mounting (11) can be inserted and held.

4. The securing device (12) according to claim 2 or 3, **characterized in that** the undercut element (14) is designed as a spring element which has one of its end portions designed so as to make a form fit with the medical container (1) and has its other end portion designed so as to be supported by sterile product mounting (11) retained in the holding section (17), thus clamping the sterile product mounting (11) in place in the holding section (17).

5. The securing device (12) according to claim 1, **characterized in that** the supporting base (16) comprises a holding section (17) to which the mounting (11) is secured or can be secured and, in a state in which the mounting (11) is secured to the container (1) by means of the securing device (12), both the undercut portion (13) and the undercut element (14) engage in recesses (3) in the base (2) substantially in the same direction and spaced from the holding section (17).

6. The securing device (12) according to claim 1 or 2, **characterized in that** the medical container (1) has a sieve-shaped or lattice-shaped base (2) and the undercut portion (13) is designed to engage around a lattice bar (6) in a form-fitting manner, whereas the undercut element (14) is designed to engage around said lattice bar (6) in a form-fitting manner from an opposite direction, in particular engaging such that the undercut portion (13) and the undercut element (14) in the secured state having a closed profile or enclosing the lattice bar (6) in a cross-sectional view/projection.

7. The securing device (12) according to one of the preceding claims, **characterized in that** the undercut portion (13) can be inserted in the first recess (3) and can be moved, in particular shifted to a securing position in which the securing section engages around the edge of the first recess (3) or the lattice bar (6) in a form-fitting manner such that the undercut portion (13) can be released from the form fit only by a movement in a preferential direction and the undercut element (14) is designed to be spring-elastic and can be made to engage in recesses (3) such that a spring force contrary to the preferential direction is applied to the securing device (12).

8. The securing device (12) according to claim 7, **characterized in that** the undercut element (14) produces a form fit transverse to the preferential direction by resting in the secured state against at least two edges of recesses (3) which in particular extend substantially parallel to the preferential direction.

9. The securing device (12) according to claim 8, **characterized in that** the undercut element (14) is designed to be fork-like and laterally engages around the undercut portion (13).

10. The securing device (12) according to one of the preceding claims, **characterized in that** the first undercut portion (13) and the undercut element (14) are arranged in a container inside (5) in the secured state and from the container inside (5) engage in the recesses (3), whereby the securing device (12) can be detached from the container (1) by an access exclusively from the container inside (5).

11. The securing device (12) according to one of the claims 1 to 10, **characterized in that** the securing device (12) forms a support area (20) which rests on the base (2) of the container (1) and the support area (20) forms at least one extension (22) which projects beyond the undercut portion (13) in a direction facing away from the holding section (16) and is adapted to have a supporting contact with the base (2) such that tilting of the securing device (12) is prevented.

12. A mounting device (10) for arranging/receiving sterile products or products to be sterilized in the interior (5) of a medical container (1), in particular a sterilization mesh basket, which comprises a base (2) having a number of recesses (3), comprising
- a sterile product mounting (11) for holding and/or receiving sterile products or products to be sterilized,
- at least two securing devices (12) according to one of the claims 1 to 11, **characterized in that**
the sterile product mounting (11) can be releasably secured without tools at each of its end faces with a securing device (12) by form-fit, and the securing devices (12) are adapted to receive different sterile product mountings (11) which for their part are adapted to receive various sterile products.

13. A modular mesh basket system for arranging/receiving sterile products or products to be sterilized, comprising
- a mesh basket (1), in particular a sterilization mesh basket, comprising an interior (5) defined by a base (2) and side walls (4) and having a number of recesses (3) in the base (2); and
- a number of mounting devices (10) according to claim 12, which can be brought into engagement with the recesses (3) and are built up in modular fashion from sterile product mountings (11) and securing devices (12) according to one of the claims 1 to 11,
**characterized in that**
for individually loading the mesh basket (1), the securing devices (12) can be made to engage different recesses (3) in a flexible manner and are adapted to receive/immobilize various sterile product mountings (11) which for their part are adapted to receive various sterile products.

## Revendications

1. Dispositif de fixation (12) d'un support de produits stériles (11) de préférence en forme d'entretoise pour sa fixation dans un récipient médico-technique (1), en particulier un panier tamis de stérilisation, comprenant :
un socle de stockage/d'accueil (16) pour le stockage/l'accueil du support de produits stériles (11), qui présente une section à contre-dépouille (13) agissant dans une première direction comme un appui fixe, qui est conçu pour être amené avec une contre-dépouille correspondante au niveau du récipient médico-technique (1) à l'état encliqueté,
un élément à contre-dépouille (14) maintenu directement ou indirectement de manière mobile au niveau du socle de stockage/d'accueil (16) comme un palier libre, qui agit dans une seconde direction orientée de manière opposée à la direction d'action de la section à contre-dépouille et est conçu pour être amené avec une contre-dépouille correspondante au niveau du récipient médico-technique (1) à l'état encliqueté et
un élément de ressort ou une section de ressort (14) au niveau de l'élément à contre-dépouille (14) ou au niveau du socle de stockage/d'accueil (16), lequel est conçu pour solliciter dans la seconde direction l'élément à contre-dépouille (14) en appui direct ou indirect au niveau du socle de stockage/d'accueil, **caractérisé en ce que**
la section à contre-dépouille (13) est en forme de U ou de crochet et est conçue pour entourer par engagement positif un bord d'un premier évidement prévu au niveau du fond (2) du récipient (1), et
l'élément à contre-dépouille (14) est conçu pour entourer par engagement positif un bord du premier évidement (3) et/ou au moins d'un second évidement (3) d'une direction opposée,
au moins l'une des deux sections de fixation (13, 14) étant conçue élastiquement.

2. Dispositif de fixation (12) selon la revendication 1, **caractérisé par** une section de maintien (17) au niveau du socle de stockage/d'accueil (16), laquelle est conçue pour maintenir par engagement positif le support de produits stériles (11) et laquelle est conçue de telle manière que le support (11), lorsque le socle de stockage/d'accueil (16) est fixé au fond (2) du récipient (1), peut être introduit perpendiculairement à la direction d'extension du fond (2) dans la section de maintien (17).

3. Dispositif de fixation (12) selon la revendication 2, **caractérisé en ce que** la section de maintien (17) est conçue comme une enveloppe en tôle, le support de produits stériles (11) pouvant être inséré et maintenu entre ses branches sensiblement en forme de U.

4. Dispositif de fixation (12) selon la revendication 2 ou 3, **caractérisé en ce que** l'élément à contre-dépouille (14) est conçu comme un élément de ressort, lequel est conçu au niveau de l'une de ses sections terminales pour entrer en engagement positif avec le récipient médico-technique (1) et prend appui avec son autre section terminale au niveau du support de produits stériles maintenu dans la section de maintien (17) et celui-ci se coince donc dans la section de maintien (17).

5. Dispositif de fixation (12) selon la revendication 1, **caractérisé en ce que** le socle de stockage (16) présente une section de maintien (17), à laquelle le support (11) est fixé ou peut être fixé et aussi bien la section à contre-dépouille (13) qu'également l'élément à contre-dépouille (14), dans un état dans lequel le support (11) est fixé au moyen du dispositif de fixation (12) au niveau du récipient (1), entrent en prise dans le fond (2) sensiblement dans la même direction que la section de maintien (17) espacée dans des évidements (3).

6. Dispositif de fixation (12) selon la revendication 1 ou 2, **caractérisé en ce que** le récipient médico-technique (1) présente un fond (2) en forme de tamis ou de treillis et la section à contre-dépouille (13) est conçue pour entourer par engagement positif une entretoise en treillis (6), alors que l'élément à contre-dépouille (14) est conçu pour entourer par engagement positif l'entretoise en treillis (6) d'une direction opposée, en particulier pour l'entourer de telle sorte que la section à contre-dépouille (13) et l'élément à contre-dépouille (14) présentent à l'état fixé dans une vue en coupe transversale/projection un profilé fermé ou renferment l'entretoise en treillis (6).

7. Dispositif de fixation (12) selon l'une quelconque des revendications précédentes, **caractérisé en ce que** la section à contre-dépouille (13) peut être insérée dans le premier évidement (3) et peut être amené, en particulier peut être déplacé, dans une position de fixation, dans laquelle la section de fixation entoure par engagement positif le bord du premier évidement (3) ou l'entretoise en treillis (6) de telle sorte que la section à contre-dépouille (13) peut se détacher uniquement par un mouvement dans une direction préférentielle de l'engagement positif et l'élément à contre-dépouille (14) est conçu élastiquement et peut être mis en prise avec des évidements (3) de telle sorte que le dispositif de fixation (12) est soumis à une force élastique contraire à la direction préférentielle.

8. Dispositif de fixation (12) selon la revendication 7, **caractérisé en ce que** l'élément à contre-dépouille (14) crée un engagement positif transversalement par rapport à la direction préférentielle, en reposant à l'état fixé au niveau d'au moins deux bords d'évidements (3), en particulier s'étendant sensiblement parallèlement à la direction préférentielle.

9. Dispositif de fixation (12) selon la revendication 8, **caractérisé en ce que** l'élément à contre-dépouille (14) est conçu en forme de fourche et entoure latéralement la section à contre-dépouille (13).

10. Dispositif de fixation (12) selon l'une quelconque des revendications précédentes, **caractérisé en ce que** la première section à contre-dépouille (13) et l'élément à contre-dépouille (14) sont disposés à l'état fixé dans un côté intérieur du récipient (5) et entrent en prise à partir du côté intérieur du récipient (5) dans les évidements (3), moyennant quoi le dispositif de fixation (12) peut être détaché du récipient (1) par un accès exclusivement à partir du côté intérieur (5).

11. Dispositif de fixation (12) selon l'une quelconque des revendications 1 à 10, **caractérisé en ce que** le dispositif de fixation (12) forme une surface d'appui (20), laquelle repose sur le fond (2) du récipient (1) et la surface d'appui (20) forme au moins un prolongement (22), lequel fait saillie dans une direction éloignée de la section de maintien (16) par l'intermédiaire de la section à contre-dépouille (13) et est adapté à un contact avec le fond (2), prenant appui par rapport à une inclinaison du dispositif de fixation (12).

12. Dispositif de support (10) pour la disposition/le stockage de produits stériles ou à stériliser dans un espace interne (5) d'un récipient médico-technique (1), en particulier d'un panier tamis de stérilisation, lequel présente un fond (2) avec un certain nombre d'évidements (3), comprenant
- un support de produits stériles (11) pour conserver et/ou stocker des produits stériles ou à stériliser,
- au moins deux dispositifs de fixation (12) selon l'une quelconque des revendications 1 à 11,
**caractérisé en ce que**
le support de produits stériles (11) peut être fixé de manière amovible par engagement positif sans outil au niveau de chacune de ses faces frontales avec respectivement un dispositif de fixation (12), et les dispositifs de fixation (12) sont adaptés pour accueillir différents supports de produits stériles (11), lesquels sont adaptés eux-mêmes respectivement pour la disposition/le stockage de différents produits stériles.

13. Système de panier tamis modulaire pour la disposition/le stockage de produits stériles ou à stériliser comprenant
- un panier tamis (1), en particulier un panier tamis de stérilisation, avec un espace interne (5) défini par un fond (2) et des parois latérales (4) et un certain nombre d'évidements (3) dans le fond (2) ; et
- un certain nombre de dispositifs de support (10) selon la revendication 12, lesquels peuvent être mis en prise avec les évidements (3) et sont constitués de manière modulaire à partir des supports de produits stériles (11) et des dispositifs de fixation (12) selon l'une quelconque des revendications 1 à 11,
**caractérisé en ce que**
les dispositifs de fixation (12) peuvent être mis en prise de manière flexible par rapport à un équipement individuel du panier tamis (1) avec différents évidements (3) et sont adaptés pour accueillir/fixer différents supports de produits stériles (11), lesquels sont adaptés eux-mêmes respectivement pour la disposition/le stockage de différents produits stériles.
